# EUROPEAN PATENT APPLICATION

(11) **EP 2 653 864 A1**
(43) Date of publication of application: **23.10.2013**
(21) Application number: 12002680.2
(22) Date of filing: 17.04.2012
(51) Int. Cl.: G01N 33/38, B62D 57/00, B62D 57/024, G01N 17/02

(54) **Climbing robot for corrosion monitoring and sensor for potential mapping**

(71) Applicant: ETH Zurich, 8092 Zürich (CH)
(72) Inventor: Leibbrandt, Alexis, 8047 Zürich (CH); Elsener, Bernhard, 8803 Rüschlikon (CH); Flatt, Robert, 8706 Meilen (CH); Siegwart, Roland, 6430 Schwyz (CH); Hürzeler, Christoph, 8304 Wallisellen (CH); Caprari, Gilles, 6900 Lugano (CH)

(57) **Abstract**

A climbing robot (20) for corrosion monitoring of reinforced concrete structures (10) comprises a robot device (30; 40) adapted to move on an essentially plan or slightly curved concrete surface independent from the orientation of the surface, especially vertically, inclined or upside down, and at least one sensor (50) for detecting properties of the concrete surface (15) the corroding areas of a reinforced concrete structure (10). The sensor comprises a wheel electrode (50; 55) adapted to detect an electrode electrical potential difference between the reinforced concrete structure (10) and a reference electrode (55) of the wheel electrode.

## Description

### TECHNICAL FIELD

The present invention relates to a climbing robot for corrosion monitoring of reinforced concrete structures comprising: a robot device adapted to move on an essentially plan or slightly curved concrete surface independent from the orientation of the surface, especially inclined, vertical and upside down, and one or more sensors for detecting properties of the concrete surface, especially detect corroding areas of a reinforced concrete structure, and further related to a sensor for potential mapping.

### PRIOR ART

Corrosion of steel in concrete is a major problem for reinforced structures. Indeed, most of the developed countries, for which a large part of the infrastructure was built before concrete technology had matured, face nowadays the impacts that this phenomenon has on aging structures.

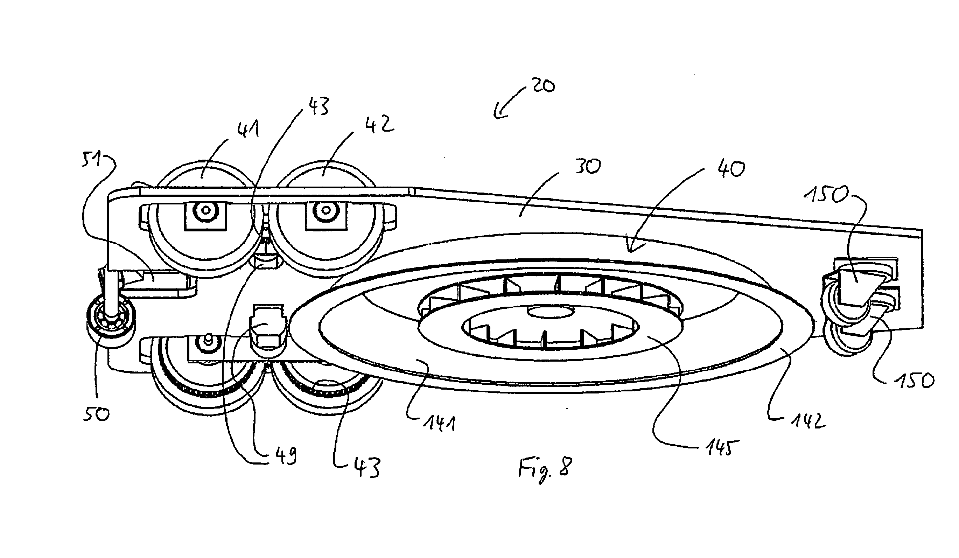

Hanyang University in Korea has developed robots for condition assessment of bridges. In 2008, Jong She Lee at al. published a paper "Advanced Robot System for Automated Bridge Inspection and Monitoring" presented at the 4^{th} International Conference on Advances in Structural Engineering and Mechanics (ASEM'08) in Jeju, Korea, May 26-28, 2008. The wall-climbing robot from Fig. 8 of said prior art has a motor-impeller type adhesion mechanism. This robot is capable to move over inclined surfaces and especially in a turn-over situation. However, that robot was intended to be used with a machine vision system to inspect the structure only visually and did not provide any potential mapping capabilities. It is also suggested there to use another technique to develop the adhesion mechanism as vacuum pad adhesion. The robots shown in this paper can be equipped with so called NDT-sensors, i.e. test sensors providing non-destructive tests as ultrasonic sensors, eddy-current measurements and ACFM.

Jörg Köhnen, Frank Weise and Herbert Wiggenhauser have published at the IABSE Symposium Melbourne 2002 within the article "A climbing robot for the assessment of civil infrastructures" a climbing robot for corrosion monitoring of reinforced concrete structures comprising: a robot device adapted to move on an essentially plan or slightly curved concrete surface independent from the orientation of the surface, especially vertically, and one or more sensors for detecting properties of the concrete surface, especially detect corroded areas of a reinforced concrete structure, by visual inspection only. A climbing robot with the features of the preamble of claim 1 is known from this document.

A motor-impeller type adhesion mechanism also called a vortex mechanism is disclosed in the patent US 6,881,025 B2 describing a suction cup vortex attractor, being able to move over inclined and turn-over situations.

It is further known from prior art to use an electrolyte potential measuring apparatus comprising a wheel electrode system from US 4,584,530 dated from Apr. 22, 1986. This document discloses a wheel electrode for potential measurements having a chamber where the electrolytic solution is placed. This potential measuring apparatus has to be pushed by the user over the surface to be measured and a cable has to be connected to the reinforcement of the concrete. The advantage of this device according to the prior art is the possibility to take continuous measurements, while the device is moving, wherein the resolution is tightly connected to the electrode sensor of the wheel electrode.

As mentioned above corrosion of steel in concrete is a major problem for reinforced structures (cooling towers, bridges, pipelines, buildings etc.). Indeed, most of the developed countries, for which a large part of the infrastructure was built before concrete technology had matured, face nowadays the impacts that this phenomenon has on aging structures.

In cold climates, one specific issue is chloride-induced corrosion, since de-icing salts (based on chlorides) are used in winter and structures in coastal zones are subjected to sea water. Chloride-induced corrosion is particularly dangerous, as it results in severe local loss of the reinforcement cross-section and frequently cannot be detected by visual inspection of the concrete surface. The only way to assess chloride-induced corrosion properly is by electrochemical methods as half-cell potential mapping, e.g. according to a methods as shown in the above-mentioned US 4,584,530. This technique, described in an ASTM standard (ASTM C876 - 09 for Standard Test Method for Corrosion Potentials of Uncoated Reinforcing Steel in Concrete) in an international RILEM recommendation, RILEM TC 154 EMC, consists in measuring the electrical potential difference between the reinforcement and a reference electrode placed on the surface of the concrete (see B. Elsener, C. Andrade, J. Gulikers, R. Polder, M. Raupach in Half-cell potential measurements - potential mapping on reinforced concrete structures. RILEM TC 154 EMC. Materials and Structures 36 (2003) 461 - 471). Areas where corrosion takes place show much lower potential values with respect to non-corroding (passive) areas. This technique is very reliable but time consuming and especially faces problems of accessibility.

### SUMMARY OF THE INVENTION

Based on this prior art it is an object of the present invention providing a climbing robot comprising a dedicated sensor for corrosion monitoring allowing for continuous measurements along the robot trajectory; and especially this can relate to measurements at a given location and over time.

This and further objects of the invention are reached with the features of the characterizing part of claim 1, wherein one sensor comprises a wheel electrode adapted to electrolytically contact the concrete surface and a reference electrode connected to the wheel to detect an electrical potential difference between the reinforced concrete structure and the reference electrode of each wheel electrode.

The present robot provides a novel technique for condition assessment of reinforced structures such as cooling towers, chimneys, dams, bridges or tunnels. More precisely, the state of steel corrosion in reinforced concrete is assessed by means of a climbing robot. The latter uses potential mapping to detect the areas with corroding reinforcement in the concrete. The sensor comprising the wheel electrode in contact with the concrete and a reference electrode is used to read the potential at the surface of the reinforced structure in a rapid and continuous way along the trajectory of the robot. This will have major benefits for society in terms of guaranteeing a much high safety and reducing the maintenance costs.

The use of a free climbing robot to perform half-cell potential mapping of any concrete surface and hence detect corroding areas will overcome the time, cost and accessibility limitations. It will make it possible to detect corrosion at a very early state, which then greatly reduces maintenance costs if adequate measures are taken. Regular application of this inspection technique will improve asset management of structures and guarantee structural safety in a quick and efficient way.

The invention comprises a specific lightweight sensor placed on the robot to take the potential readings. The sensor is based on a dedicated wheel electrode system. It is a further advantage of the sensor on the robot according to the invention allowing the robot to take continuously measurements while moving on the concrete surface. Data are preferably transmitted to a base station for postprocessing in the same way as for traditional half-cell potential mapping (potential maps, isopotential graphs or statistical evaluation). This enables the responsible of the condition assessment to locate the places where corrosion is on-going and to follow its evolution with time.

More precisely, the electrode system is formed by the reference electrode, a contact wheel filled with electrolyte, comprising water, and a tube making the connection between these two. The tube is connected to a peristaltic pump allowing the contact wheel to perform a constant and adjustable electrolytic contact with the concrete cover.

The robot according to the invention uses the impeller technology known from US 6,881,025 B2 and has the advantage of being able to climb on vertical surface or to move upside-down. This is a main advantage regarding to traditional inspection techniques as it can access to large structures without the need of a scaffold. The main principle is based on a vortex generated by the robot, which creates a low-pressure region between robot and the surface and with that delivers an adhesion force. However, it is not mandatory to use the vortex method to create the adhesion force of the robot.

The climbing robot according to an embodiment of the invention comprises a vortex attraction means for generating a recirculating vortex flow and a low pressure region resulting therefore attracting the robot device towards the surface. The climbing robot furthermore and preferably comprises at least two further wheels adapted to contact the concrete surface as well, wherein at least one wheel is driven. These three wheels, preferably attached in a triangle on a frame of the robot allow overcoming concrete irregularities, small gaps, small holes etc.

Preferably the group of wheels are positioned on the ends of an axis being their rotation axis or an axis parallel thereto, If this axis is then positioned centrally (i.e. perpendicular) behind the impeller in the direction of normal movement of the robot, wherein the wheel electrode is positioned centrally on the other side of said axis, these four elements span a rhomb allowing for small scale turns, especially if the distance of the centre of the impeller to the axis is greater than the distance of the axle of the wheel electrode to the axis.

The robot device can be radio-controlled, follow a pre-programmed path or follow a path calculated in real-time through learning the surface dimensions, features and structure.

Further embodiments of the invention are laid down in the dependent claims.

A sensor for potential mapping to detect areas of corroding reinforcement is also disclosed within the scope of the invention comprising: a wheel performing a constant electrolytic contact with the surface, a reference electrode connected to the wheel at a given distance through a electrolyte conduit, wherein the wheel comprises an outer wettable surface and the electrolyte conduit is contacting the wettable surface through the hub of the wheel and electrolyte paths from the hub to the wettable surface.

Providing radial cavities in the wheel connecting the central partly hollow hub with the wettable surface are very efficient electrolyte paths. They can be provided as two, three, four or more electrolyte paths in regular angular distance one from the other (seen in the direction of the axis of rotation of the wheel.

Preferably, a electrolyte feeding tube is connected with the reference electrode at the beginning of the electrolyte conduit and the reference electrode is removably connected at the fluid connector between the beginning of the electrolyte conduit and the electrolyte feeding tube. This allows a free replacement of the electrode without dismounting the electrode wheel. Additionally this sequence of feeding tubes allows continuous pumping and providing electrolyte, comprising mostly water, from a reservoir to the electrode and wheel. The liquid in the electrolyte tube is provided with enough pression to expulse the "air bubbles" to the outer layer of the wheel.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows schematical perspective view from below a wall on which a robot with sensor according to an embodiment of the invention is moved,
- Fig. 2: shows a partial cross-section of the robot from Fig. 1 on a wall with supply and measurement connections;
- Fig. 3: shows a schematical perspective view from below a wall on which the robot with sensor according to the embodiment shown in Fig. 1 and 2 is attached,
- Fig. 4: shows a schematical view from above on the robot with sensor according to the embodiment shown in Fig. 1 and 2,
- Fig. 5: shows a schematical side view on the robot with sensor according to the embodiment shown in Fig. 1 and 2,
- Fig. 6: shows a schematical perspective view onto the sensor assembly according to the embodiment of the robot shown,
- Fig. 7: shows a cross-section schematical view onto the sensor assembly according to Fig. 6, and.
- Fig. 8: shows a schematical view from below on the robot with sensor according to the embodiment shown in Fig. 1 and 2.

### DESCRIPTION OF PREFERRED EMBODIMENTS

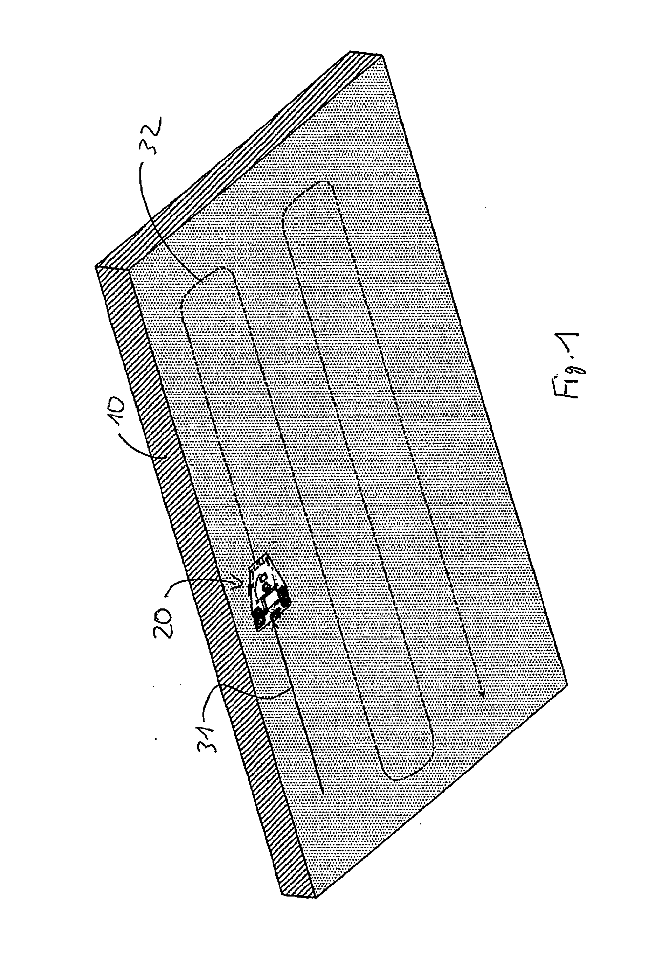

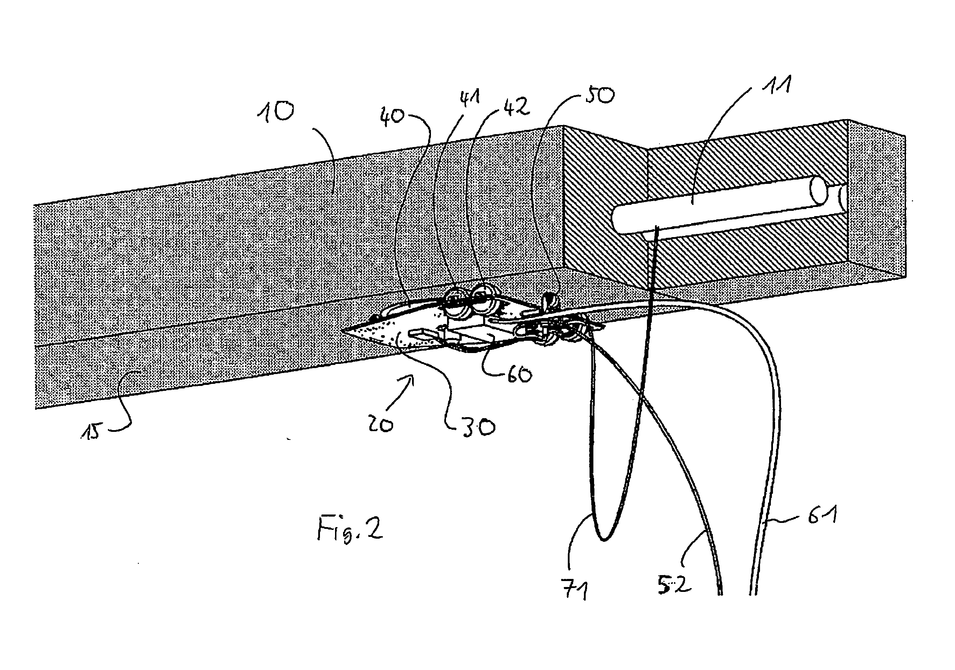

Fig. 1 shows schematical perspective view from below a wall 10 on which a robot 20 with sensor according to an embodiment of the invention is moved. The travelled path 31 will be extended into path 32 covering the entire surface of the wall, being a concrete wall with reinforced steel structure. Fig. 2 now shows a partial cross-section of the robot 20 from Fig. 1 on a wall 10 with supply and measurement connections 52, 61 and 71 as explained below.

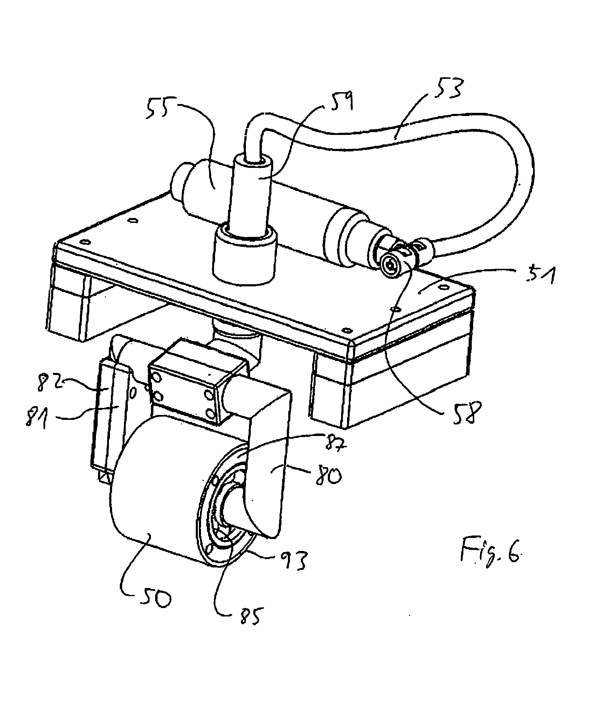

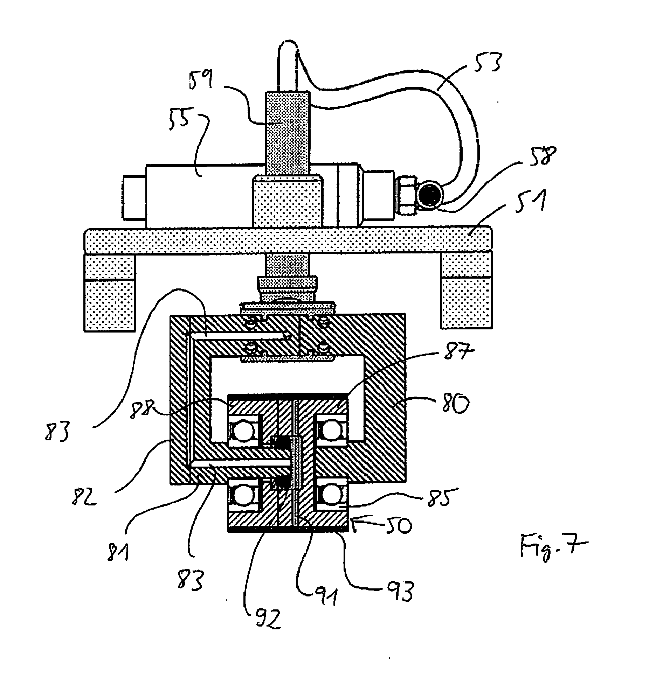

The robot 20 attaches itself on the more or less smooth hard surface 15 of the concrete wall with the help of an impeller vortex device 40. Said device can be built following the teaching of US 6,881,025 B2, allowing to be moved, as shown in Fig. 1 in a turn-over situation. The robot 20 comprises preferably a frame plate 30 onto which drives and control units are provided as well as supply and measurement connections 61, 52 and 71. Power converter 60 is connected with the environment with a power supply line 61 also comprising control lines for steering the drives. The control can also be achieved through a radio link. Furthermore a water tube 52 connects the electrode 55, especially shown in Fig. 6 and 7, with a remote control unit, reading all data received from the electrode 55. The reinforced steel structure 11 is attached via an elongate conductor 71 with the respective control unit, preferably on the frame 30. Usually the elongate conductor 71 is guided to the user and is then prolonged from there to a steel structure 11 of the concrete wall 10.

Driven wheels 41 and 42 are integrated into the frame 30 as will be explained below. The impeller assembly 40 receives a continuous power supply via the power line 61 to be able to remain attached on inclined or ceiling surfaces. The impeller diameter decides together with the distance between the impeller and the wall surface or the length of the impeller's skirt about the possible curvature of the surface 15. It is also possible to integrate a buffer battery into the frame 30.

As will be explained below, the sensor 50 needs a supply of water; this can be realized through a water reservoir on the frame 30, which also can be a buffer reservoir, being fed through line 52 from the place of the user, having a water reservoir with a peristaltic pump to pump the water upwards to the electrode roller 50 after having passed the contct to the electrode 55.

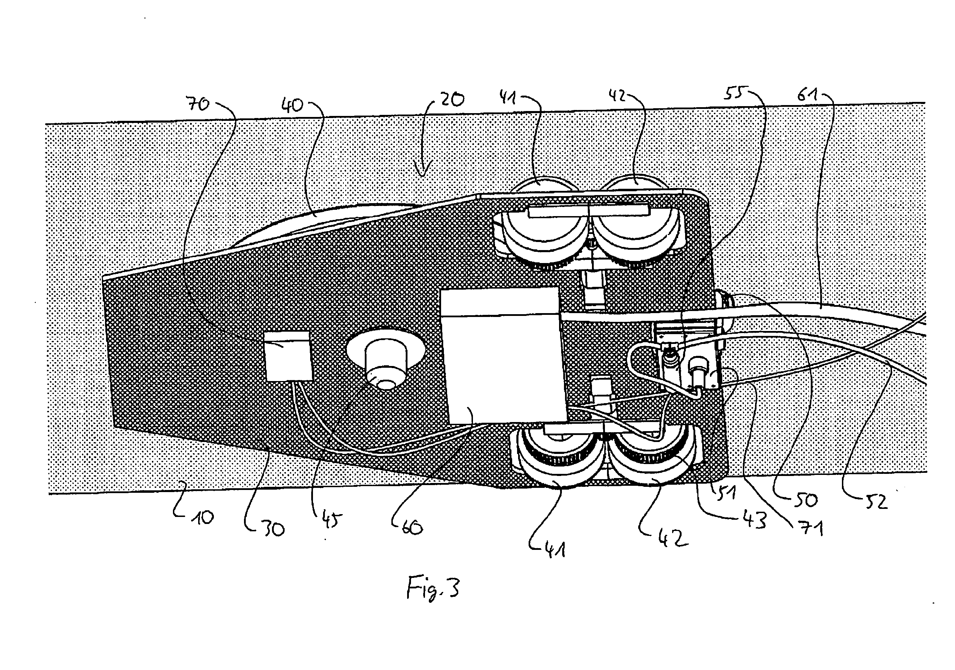

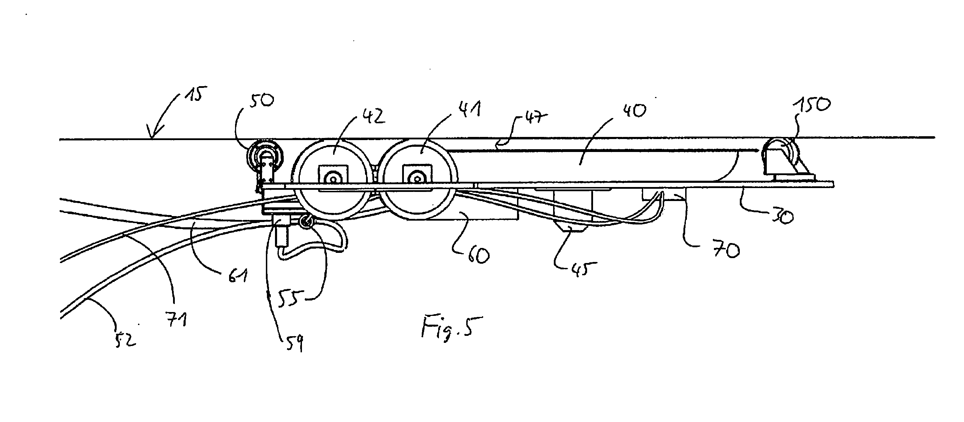

Fig. 3 shows a schematical perspective view from below a wall 10 on which the robot 20 with sensor 50 according to the embodiment shown in Fig. 1 and 2 is attached. Identical or similar features have received the same and identical reference numerals throughout the specification. It can be seen in Fig. 3 that frame 30 is trapezoidal in the direction of movement, where caster wheels 150 (not visible in Fig. 3) provide support at the front side. Impeller assembly 40 attached at impeller holding element 45 is attached near the centre of gravity of the robot 20 in order to receive mainly forces in direction of the impeller main axis. Near the holding element 45, electrode control unit 70 and power transformer 60 are attached on the frame 30. Electrode control unit 70 can be used not only for the electrode potential sampling but for the impeller and the driving system also, e.g. in connection with the power transformer. In the rectangular back part of the frame 30 two sets of two driven rollers 41 and 42 are provided, which can be directly coupled via a gear wheel mesh assembly 43. The height of rollers 41 and 42 are essentially the same and at least as high as the lower surface of the impeller (see Fig. 5 for gap 47) of impeller assembly 40.

Driving wheels 41 and 42 are mounted on parallel axes one behind the other which allows a flexibility to operate on rounded surfaces 15 and to overcome small irregularities in the concrete surface (holes, small gaps). A slightly curved concrete surface 15 is defined as a surface 15, which has over the length of the frame 30 of the robot 20 a greatest elevation or depression over the plan surface of less than 10% of the length of the frame 30 of the robot 20.

In the middle of the back side of frame 30, one sensor roller 50, having essentially the same height than driven rollers 41, 42 and two caster wheels 150 are attached to frame 30 via a sensor plate 51. Two caster wheels 150 mounted side by side allow an improved turning of this forward end of the frame 30 through rotation of the backwards mounted driven wheels 41 and 42.

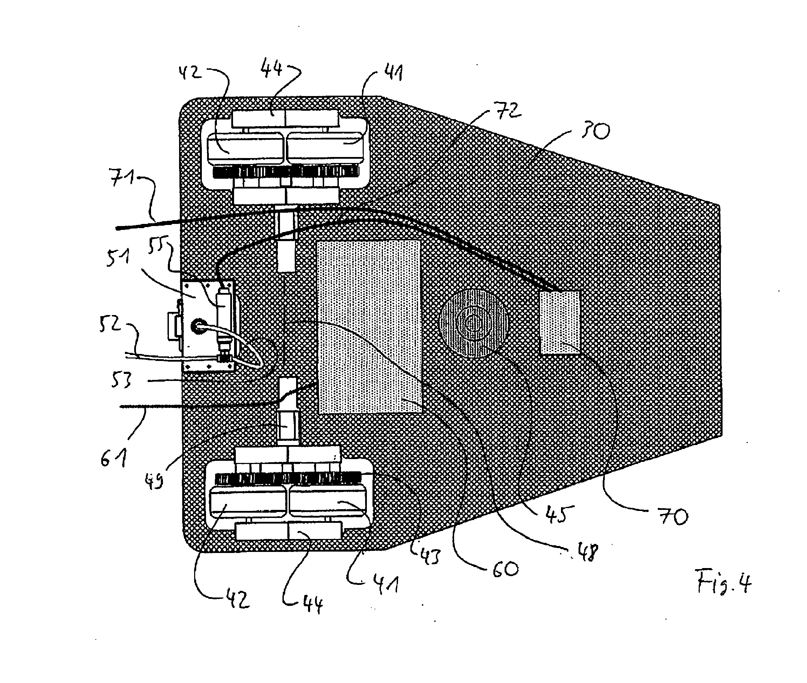

Fig. 4 shows a schematical view from above on the robot 20 with sensor assembly 50 according to the embodiment shown in Fig. 1 and 2. It can be seen in addition to Fig. 3 that the elongate conductor 71 is connected with the electrode control unit 70 and is then guided as conductor line 72 to the electronic output of electrode body 55. In other words; the potential signal from the electrode 55 is combined with the potential signal from the reinforced structure via line 71 in control unit 70; although it is clear that the measurement unit can also be realized in the hands of the user where lines 71, 52 and 61 come together.

At least one of driven rollers 41 and 42 of each pair of rollers 41 and 42 is driven through a drive motor, and the other roller of the pair can be driven connected via gear rollers 43. Reference numeral 44 points to the holding structure of the wheel assembly. The motor is located on the other side of the holding structure 44 and is in fact connected to the small gear which is then connected to the two bigger gears 43 of both wheels 41, 42. It is preferred that the control lines (not shown) connecting the drive with the user (or the control unit 70) also comprise encoder information about the position of the rollers 41, 42 or a specific position sensor is provided together with the rollers 41, 42. The device is turned through stopping or retrograding one pair of rollers 41, 42 and moving the other two rollers 41 and 42, so that the turning point of the frame is near or between the roller pairs 41, 42, which allows turning the frame, while sensor roller 50 remains essentially at the same place, thus allowing taking measurements at the same place while turning the robot 20. In other words, it is adapted for continuous measurements leading to surface mapping not only on straight portions. In order to improve this result, the group of wheels 41, 42 are positioned on the ends of an axis being their rotation axis or an axis parallel thereto 48, wherein this axis 48 is positioned centrally behind the impeller (Fig. 4 shows the impeller cover with reference numeral 40 as part of the impeller system) in the direction of normal movement of the robot 20. On the other hand, the wheel electrode 50 is positioned centrally on the other side of said axis 48, wherein preferably the distance of the centre of the impeller to the axis 48 is greater than the distance of the axle 59 of the wheel electrode to the axis 48.

Fig. 5 shows a schematical side view on the robot 20 with sensor 50 according to the embodiment shown in Fig. 1 and 2 on the surface 15 of a wall 10, attached from below through underpressure generated by impeller assembly 40 even with a large gap 47. In an alternative embodiment a semi rigid skirt can be attached to the underside of the impeller to increase the adhesion of the impeller system 40 or to enable the use of a completely different adhesion principle like vacuum.

Fig. 8 shows a schematical view from below on the robot 20 with sensor 50 according to the embodiment shown in Fig. 1 and 2. Wheel drive motors 49 are attached below the frame 30 and drive rollers 41 and 42 through the central small gear roller 43. It is also possible to provide one or two direct drives for the rollers 41, 42.

It can be seen that beside the two front caster wheels 150 (front in the sense of the usual motion of the robot 20), the electrode wheel 50 is also mounted as a caster wheel. It is also possible to provide two or more electrode wheels 50, e.g. one or two in the rear area between rollers 41 and e.g. one electrode wheel 50 in the front area of frame 30 adjacent to or replacing a caster wheel 150.

The vortex system 40 comprises an impeller 145 centrally mounted in a skirt or cover 141 having in the present embodiment a lower horizontal surface 142, being positioned in a gap 47 over a possible surface 15. The existence of the gap and structure of the skirt or cover 141 depend on the embodiment of the vortex system employed. It is important, that the suction effect is continuous and allows rolling of the wheels 150, 42, 41 and of course 50 on the surface to be inspected, allowing for a continuous measurement process and or direct displacement of the robot 20 to a specific place to be inspected.

Fig. 6 shows a schematical perspective view onto the sensor assembly according to the embodiment of the robot 20 shown, and Fig. 7 shows a cross-section schematical view onto the sensor assembly according to Fig. 6. Sensor plate 51 is attached to frame 30 of robot 20. It is also possible to mount the different elements as explained below directly on the frame 30.

A hole through plate 51 allows introducing main axle 59 of the sensor assembly, allowing to rotate the sensor roller 50 around an axis perpendicular to plate 51. Electrode body 55 is positioned and fixed on plate 51. Electrode body 55 is connected via electrolyte line 53 to the main axle 59 and connector 58 allows attaching fluid tube 52 to the electrode body.

Main axle 59 comprises a L-shaped lower end for attaching a bifurcating element. Above the L-shaped portion and between a flange above that portion and the lower surface of the plate 51 is preferably provided a spring, e.g. a pressure spring provided around the body of axle 59 to ensure that the electrode roller 50 is always pushed towards the surface 15 to cope with the irregularities of the surface 15 independently from the position of the frame 30. This is especially an advantage, if the robot moves along a slightly curved surface, and especially a convex surface.

Main axle 59 bifurcate into a first full wheel shaft part 80 and a second hollow wheel shaft part 81. Said second shaft part 81 is covered by a cover 82. The c-shaped shaft parts 81 and 82 are then re-joined on the common rotation axis of the roller 50. Roller 50 is connected with shaft parts 81 and 82 via ball bearings 85. Axes of elements 80, 81 and 82 are parallel at a given distance of axis 59 in order to have a proper caster wheel compared to caster wheels 150.

The cross section of Fig. 7 shows the fluid conduit starting from fluid connection tube 53, going through axle 59 entering water conduits 83 in hollow wheel shaft part 81, being covered through cover 82 and contacting water path element 91 in the roller 50. In a different embodiment, the water path could also be provided in both halves of the shaft 80, 81.

Roller 50 comprises a soft moist and soaked roller surface 93 covering the two wheel body parts 87 and 88. The wheel body parts 87 and 88 are positioned around the rotation axis of the roller 50 through roller bearings 85 on their outer side. Within the wheel body part 87, covering more than half of the wheel 50 at least one water path 91 is provided. In fact, beside these two paths 91 also a different number of paths could be provided, e.g. four paths in 90 degree separation, six paths 60 degree separation, eight paths with 45 degree separation etc., if looked upon in the direction of the rotation axis of the roller 50. Water enters from the conduit 83 into water path 91 and is blocked to leave the roller at the hub through a joint 92. The water path 91 can be an empty cavity or filled with a water-carrying material. The water is well distributed along the short water path 91 at a greater area of roller surface 93 ensuring continuous wetting of the contacting wheel with the pump and unlimited measurements in time (limited only by the user reservoir) with and adjustable flow of water to the contacting wheel electrode according to the concrete humidity and/or the speed of the robot 20 and a homogeneous distribution of the water around the outer part surface 93 of the sensor wheel 50.

The surface 93 of the roller provides the electrode surface as e.g. known and used in US 4,584,530. Water is continuously fed to the surface to ensure a good electrolytic contact between the roller 50 and the electrolytic being surveyed. A difference between the sensor of said US 4,584,530 and the present embodiment lies in the fact that the metal reference electrode in contact with the electrolytic solution for sensing voltage potentials is provided in a distance in the electrode body 55. This however, does not influence the measurement in a detrimental way, since the water and electrolytic connection between the electrode in body 55 and the surface to be surveyed near roller surface 93 is ensured through a continuous water flow provided through conduit 52 and filled tube 53. It is even faster to replace the reference electrode 55 without removing the wheel 50. The solution is lightweight, since the electrode elements can be provided outside the sensor wheel 50. The electrode 55 is not contaminated by the concrete surface 15. No copper ions are released to the concrete surface, since the distance from the copper reference electrode to the surface 15 is about at least 10 centimeters.

A prototype of the climbing robot 20 according to the invention for corrosion monitoring has provided successful measurements in preliminary tests on a corroded reinforced concrete block 10. The implemented prototype includes as shown in the drawings: the adhesion mechanism 40 generated by the vortex, the sensor 50 for potential mapping (wheel electrode), the locomotion mechanism 41, 42 supported by two driving wheels (two motors) and caster wheels 150, the power supply 61 through a battery or a cable from the base station to the robot, and finally control and communication functions.

The robot 20 was able to move over any concrete surface 15, independent from its orientation. Additionally it was able to move over slightly curved surfaces. Such a surface is defined as a surface, which has over the length of the frame 30 of the robot 20 a greatest elevation over or depression under the plan surface of less than 10% of the length of the frame 30 of the robot 20.

It is possible to mount on frame 30 additional sensors, like a camera, e.g. to check the aspect of the surface 15 or to drive the robot 20, or magnetic sensors (cover meter) or humidity sensors (concrete humidity).

### LIST OF REFERENCE SIGNS

- 10: concrete wall
- 11: steel reinforcement
- 15: concrete wall surface
- 20: climbing sensor robot
- 30: frame plate
- 31: travelled path
- 32: future path
- 40: impeller assembly (impeller and casing)
- 41: driven roller
- 42: driven roller
- 43: gear roller
- 44: wheel holding structre
- 45: holding element for impeller assembly
- 47: gap
- 48: middle axis of driven wheels
- 49: wheel drive unit
- 50: sensor roller
- 51: sensor plate
- 52: fluid tube
- 53: fluid connection tube
- 55: electrode body
- 58: connector
- 59: main axle
- 60: power transformer
- 61: electric supply line
- 70: electrode control unit
- 71: elongate conductor
- 72: conductor line
- 80: wheel shaft part
- 81: wheel shaft part
- 82: wheel shaft cover
- 83: water conduit
- 85: ball bearing
- 87: wheel body part
- 88: wheel body part
- 91: water path
- 92: j oint
- 93: elastic moist material roller cover
- 141: impeller cover / skirt
- 142: lower cover surface
- 145: impeller
- 150: front roller / caster wheels

## Claims

1. A climbing robot (20) for corrosion monitoring of reinforced concrete structures (10) comprising:
- a robot device (30; 40) adapted to move on an essentially plan or slightly curved concrete surface (15) independent from the orientation of the surface, especially vertically, inclined or upside down,
- at least one sensor (50) for detecting properties of the concrete surface (15) the corroding areas of a reinforced concrete structure (10),
**characterized in that** one sensor comprises one or more wheel electrodes (50; 55) adapted to detect an electrical potential difference between the reinforced concrete structure (10) and a reference electrode (55) of the wheel electrode.

2. The climbing robot (20) according to claim 1, wherein the robot device (30; 40) comprises a vortex attraction means (40) for generating a recirculating vortex flow and a low pressure region resulting therefore attracting the robot device (20) towards the surface (15).

3. The climbing robot (20) according to claim 2, wherein a control unit (70) of the wheel electrode(s) is adapted to allow a continuous measurement of the potential difference along the trajectory of the robot (20) during movement of the robot.

4. The climbing robot (20) according to any one of claims 1 to 3, wherein the robot device (30, 40) comprises at least two further wheels (41, 42, 150) adapted to contact the concrete surface (15) as well, wherein at least one wheel (41, 42) is driven.

5. The climbing robot (20) according to claim 4, wherein two wheels or group of wheels (41, 42) are positioned on the ends of an axis being their rotation axis or an axis parallel thereto (48), wherein this axis (48) is positioned centrally behind the vortex attraction means (40) in the direction of normal movement of the robot (20) and wherein the wheel electrode(s) (50; 55) is or are positioned centrally on the other side of said axis (48), wherein preferably the distance of the centre of the vortex to the axis (48) is greater than the distance of the axle (59) of the wheel electrode (50; 55) to the axis (48).

6. The climbing robot (20) according to claim 5, wherein one or two additional caster wheels (150) are provided at the centre of the front of the robot.

7. The climbing robot (20) according to claim 6, wherein a slightly curved concrete surface (15) is a surface (15), which has over the length (30) of the robot (20) a greatest elevation or depression over the plan surface of less than 10% of the length (30) of the robot (20).

8. The climbing robot (20) according to any one of claims 5 to 7, wherein the two wheels (41, 42) are groups of two wheels one positioned behind the other in the movement direction.

9. The climbing robot (20) according to any one of claims 1 to 8, wherein each of the wheels (50) of the electrode(s) (55) is attached to the robot device (30; 40) on the side of the surface (15) to be monitored wherein it is biased against that surface (15) by a spring means provided between the robot device (30; 40) and the wheel (50).

10. A sensor (50, 55) for potential mapping to detect corroded areas comprising:
- a wheel (50) performing a constant electrolytic contact with the surface (15),
- at least one reference electrode (55) connected to the wheel (59) at a given distance through an electrolyte conduit (53, 83, 91),
wherein the wheel (50) comprises an outer wettable surface (93) and the electrolyte conduit (53, 83, 91) is contacting the wettable surface (93) through the hub of each wheel (50) and electrolyte paths (91) from the hub to the wettable surface (93).

11. The sensor according to claim 10, wherein the electrolyte paths (91) are radial cavities in the wheel (50) connecting the central partly hollow hub with the wettable surface (93), especially two, three, four or more electrolyte paths in regular angular distance one from the other.

12. The sensor according to claim 10 or 11, wherein a water feeding tube (51) is connected with the reference electrode (55) at the beginning of the electrolyte conduit (53, 83,91).

13. The sensor according to claim 12, wherein the at least one reference electrode (55) is removably connected at the fluid connector (58) between the beginning of the electrolyte conduit (53, 83, 91) and the electrolyte feeding tube (51), preferably pumping and providing an electrolyte from a reservoir.
